# EUROPEAN PATENT APPLICATION

(11) **EP 2 236 141 A1**
(43) Date of publication of application: **06.10.2010**
(21) Application number: 10075137.9
(22) Date of filing: 26.03.2010
(51) Int. Cl.: A61K 31/713, C12N 15/113, A61P 31/22

(54) **siDNA oligonucleotide as antiviral agent against Herpes virus Infections**

(30) Priority: 01.04.2009 EP 09075162
(71) Applicant: Universität Zürich, 8006 Zürich (CH)
(72) Inventor: Mölling, Karin Prof. Dr., 14195 Berlin (DE)
(74) Representative: Boeckh, Tobias

(57) **Abstract**

The invention relates to short interfering DNA oligonucleotides (siDNA) capable of binding to Herpes virus RNA, whereby the siDNA oligonucleotides comprise of an antisense-strand that is complementary to the Herpes virus target RNA and a second strand that is partially complementary to the antisense-strand, **characterized in that** the second strand is connected to the antisense strand by a thymidine linker, preferably 4 nucleotides in length. The siDNA oligonucleotides of the present invention are intended for use as an antiviral therapeutic agent in the treatment and/or prevention of Herpes virus infection.

SEQUENCE LISTING

<110>  University of Zurich

<120>  siDNA oligonucleotides as an antiviral agent against Herpes virus
        infections

<130>  XI 287/10

<150>  EP 09075162
<151>  2009-04-01

<160>  9

<170>  PatentIn version 3.3

<210>  1
<211>  54
<212>  DNA
<213>  Synthetic

<400>  1
gttctttcgg gtcatgctct tgtaattttt taatagacaa tgaccacgtt cgga          54

<210>  2
<211>  54
<212>  DNA
<213>  Synthetic

<400>  2
tttgggtcta atcaaggcca ttcggttttc cggttggaat tgattgcgaa agtt          54

<210>  3
<211>  54
<212>  DNA
<213>  Synthetic

<400>  3
ccgttttttt catcattttc ctctgttttc agtcgaagtt gatgatgttg tacc          54

<210>  4
<211>  54
<212>  DNA
<213>  Synthetic

<400>  4
cccgttggga tggtggggat gtcccttttc ccgaatcaac accataaagt accc          54

<210>  5
<211>  54
<212>  DNA
<213>  Synthetic

<400>  5
cccgttggga tggtggggat ggcccttttc ccgaatcaac accataaagt accc          54

<210>  6
<211>  25
<212>  RNA
<213>  Herpes simplex virus 1

<400>  6
uccuaacgug gucauugucu auuaa                                          25

<210>  7
<211>  25
<212>  RNA
<213>  Herpes simplex virus 1

<400>  7
aacuuucgca aucaauucca accgg                                          25


<210>  8
<211>  25
<212>  RNA
<213>  Herpes simplex virus 1

<400>  8
gguacuucau caucaacuuc gacug                                          25


<210>  9
<211>  25
<212>  RNA
<213>  Herpes simplex virus 1

<400>  9
ggguacuuua ugguguugau ucggg                                          25

## Description

The invention relates to short interfering DNA oligonucleotides (siDNA) capable of binding to Herpes virus RNA, whereby the siDNA oligonucleotides comprise of an antisense-strand that is complementary to the Herpes virus target RNA and a second strand that is partially complementary to the antisense-strand, **characterized in that** the second strand is connected to the antisense strand by a thymidine linker, preferably 4 nucleotides in length. The siDNA oligonucleotides of the present invention are intended for use as an antiviral therapeutic agent in the treatment and/or prevention of Herpes virus infection.

### BACKGROUND OF THE INVENTION

The *Herpesviridae* are a large family of DNA viruses that cause diseases in animals, including humans. The members of this family are also known as Herpes viruses. Herpes viruses all share a common structure comprising of a relatively large double-stranded, linear DNA genome, usually encoding 100 - 200 genes. The severity of symptoms arising from Herpes virus infections can range from mild to life threatening, becoming more severe in immune-compromised individuals.

The Herpes virus family includes various viruses that share similar molecular and structural characteristics but lead to distinct pathophysiologies. Two of the most common Herpes viruses are the Herpes simplex viruses. Herpes simplex virus-1 (HSV-1) generally infects mucoepithelial cells and leads predominantly to Oral Herpes, but can also lead to genital Herpes, as well as other Herpes simplex infections. Herpes simplex virus-2 (HSV-2) also infects mucoepithelial cells and leads predominantly to genital Herpes.

The Varicella zoster virus (VZV) also infects mucoepithelial cells and causes conditions such as Chickenpox and shingles. The Epstein-Barr virus (EBV) infects B cells and epithelial cells and has been associated with a host of different diseases, such as infectious mononucleosis, and has been implicated in the causation of Burkitt's lymphoma and nasopharyngeal carcinoma. It is further suspected to have a role in the pathogenesis of chronic fatigue syndrome and multiple sclerosis.

Cytomegalovirus (CMV) generally infects monocytes, lymphocytes, and epithelial cells and can lead to Infectious mononucleosis-like syndrome and retinitis. Other Herpes virus variants include the Roseolovirus, which causes roseola infantum, and Kaposi's sarcoma-associated Herpes virus (KSHV), which is a type of rhadinovirus which can cause Kaposi's sarcoma, primary effusion lymphoma and some types of multicentric Castleman's disease.

Seroprevalence in the United States of some of the more common Herpes viruses are HSV-1: 50 - 80 %, HSV-2: 20 - 50 %, VZV: 85 - 95 %, CMV: 40 - 70 % and EBV: 80 - 95 %.

Cold sores, or oral Herpes, are very common and are caused by the Herpes simplex virus. Cold sores are small, painful, fluid-filled blisters or sores that appear on the lips, mouth, throat, cheek, chin, or nose. It is estimated that up to 80% of the US population has been exposed to the Herpes simplex virus, HSV-1.

Genital Herpes refers to virus caused by HSV-1 or HSV-2, although usually occurs due to infection by HSV-2. Genital Herpes is a contagious viral infection generally transmitted during sexual contact and primarily affects the genitals of both men and women. Genital Herpes is characterized by recurrent clusters of vesicles and lesions at the genital areas or below the waist.

HSV-1 infections are commonly treated with the guanosine analogue Aciclovir but reports of Aciclovir resistance are increasing. Due to the high frequency of infection, there is a strong demand for the development of novel anti-Herpes virus therapeutics.

Alternative approaches to treat Herpes virus infections have involved efforts to establish small interfering ribonucleic acids (siRNAs) for antiviral treatment. siRNA relates to a mechanism based on inactivating a target RNA by double stranded RNA. Here one strand of the siRNA is poly-hybridized to the target RNA by Watson Crick base pairing, the so-called antisense strand. A second RNA strand, the passenger strand, is fully complementary to the antisense strand and only transiently required for inactivation of the RNA. It is removed in a so-called RISC-complex. This mechanism of inactivation of the target RNA by siRNA is generally called silencing.

Antiviral approaches targeting Herpes viruses using siRNA are known in the prior art (Zhang YQ et al., Clin Exp Dermatol 2008, 33:56-61 and Palliser D et al., Nature 2006, 439:89-94). These studies disclose the targeting of various HSV genes using siRNA methods. The genes selected for targeting were UL5, a component of the helicase/primase complex, UL29, the single strand binding protein (SBB), UL30, the polymerase, UL48, a leaky late gene product and VP16, the tegument protein which is a component of the virion and facilitates IE gene expression in the next round of infection. HSV-2 was inhibited by siRNA targeted against UL5, 29 and 30 and HSV-1 by siRNA targeted against UL30 and UL48.

Recently, another class of short interfering nucleic acids, partially double-stranded hairpin loop-structured oligodeoxynucleotides (ODNs), has been shown to facilitate hydrolysis of HIV RNA by binding to the viral RNA. This new class of oligodeoxynucleotides consists of an approximately 25mer antisense strand, which is highly complementary to the target mRNA site, a linker, and another second strand that is partially complementary to the antisense strand. ODNs designed on these structural principles are known to mediate hydrolysis of HIV-1 RNA by HIV-1 reverse transcriptase (RT)-associated RNase H (Matzen et al., Nature Biotech. 2007, 25, 669-674). This has been shown to occur in HIV virions in patient-derived plasma in a sequence specific manner (Heinrich et al., AIDS 2009, 23:213-221).

As shown previously by the inventor, this mechanism activates the RNase H of the virus and leads to silencing of the viral RNA. The RNase H is a Hybrid-specific RNase which was discovered by Moelling et al (Nature New Biology 1971, 234, 240-243). The inventor has also observed that cellular RNase H-like activities such as RNase HI and RNase H2A, B, C and even Agonaute 2 contribute to siDNA-mediated silencing. Agonaute 2 (Ag02) is the enzyme known to induce siRNA-mediated silencing.

Furthermore, it could be shown by the inventor that siDNA is able to induce silencing of an oncogenic retrovirus, the Spleen Focus-Forming Virus and prevent infection, cause delay of disease progression and lead to increased survival time (Matzen et al., Nature Biotech. 2007, 25, 669-674). Furthermore the inventor showed in several cases that siDNA is superior to a single-stranded antisense effect (Jendis et al., AIDS Research and Human Retroviruses 1996, 12, 1161-1168, Jendis et al., AIDS Research and Human Retro-viruses 1998, 14, 999-1005, Moelling et al., FEBSLetters 2006, 580, 3545-3550, Matzen et al., Nature Biotech. 2007, 25, 669-674).

### SUMMARY OF THE INVENTION

In light of the prior art the technical problem underlying the present invention is to provide an antiviral agent for use in the prevention and/or treatment of Herpes virus infection using siDNA.

This problem is solved by the features of the independent claims. Preferred embodiments of the present invention are provided by the dependent claims.

Therefore, an object of the invention is to provide short interfering DNA oligonucleotides (siDNA) capable of binding to Herpes virus RNA, whereby the siDNA oligonucleotides comprise of an antisense-strand that is complementary to the Herpes virus target RNA and a second strand that is partially complementary to the antisense-strand, **characterized in that** the second strand is connected to the antisense strand by a thymidine linker, preferably 4 nucleotides in length. It is intended that the antisense and second strands are 20 to 35 nucleotides in length, preferably 25 nucleotides in length, and that the antisense strand is more than 80% complementary, preferably more than 90% complementary, to the Herpes virus target RNA. The second strand is 40 - 60% complementary to the antisense-strand and is capable of forming triple helices by non-Watson-Crick base pairing with the Herpes virus target RNA.

In a preferred embodiment the siDNA oligonucleotides of the present invention are intended for use as an antiviral therapeutic agent in the treatment and/or prevention of Herpes virus infection. In a further embodiment the subject matter of the invention also relates to the use of siDNA oligonucleotides according to the present invention in the manufacture of a pharmaceutical composition for use as an antiviral therapeutic agent for the treatment and/or prevention of Herpes virus infection.

In a preferred embodiment of the present invention the siDNA oligonucleotides are stabilized by base modifications, preferably by phosphorothioate modification of the DNA.

In a further preferred embodiment of the present invention the siDNA oligonucleotides comprise the DNA sequences of SEQ ID NO. 1, SEQ ID NO. 2, SEQ ID NO. 3, SEQ ID NO. 4 and/or SEQ ID NO. 5.

The siDNA oligonucleotides of the present invention are also intended in a preferred embodiment to exhibit an antisense strand that binds Herpes virus RNA corresponding to the UL5, UL29, UL30 and/or UL48 genes. In a further embodiment, the Herpes virus target RNA sequences are to be selected from the group comprising SEQ ID NO. 6, SEQ ID NO. 7, SEQ ID NO. 8 and/or SEQ ID NO. 9.

It is further intended that the siDNA oligonucleotides of the present invention bind and/or target RNA from all viruses of the *Herpesviridae* family, especially HSV-1, HSV-2, VZV, EBV, CMV, Roseolovirus and/or KSHV.

The subject matter of the present invention also relates to a pharmaceutical composition comprising siDNA oligonucleotides of the present invention capable of binding to Herpes virus RNA as an antiviral therapeutic for the treatment and/or prevention of Herpes virus infection. Such a pharmaceutical composition or agent can be prepared such that different siDNA oligonucleotides directed to different RNA targets may be combined in a cocktail.

The pharmaceutical composition of the present invention can also be combined with a pharmaceutically acceptable carrier to form a microbicidal composition for the treatment and/or prevention of Herpes virus infection.

In a further preferred embodiment, the pharmaceutical composition in the form of a microbicidal composition can be applied topically, preferably rectally and/or to the genitals, preferably to the vagina. It is intended that the siDNA-containing pharmaceutical composition is applied to the skin, particularly the skin areas of the lips, mouth, nose, chin or eyes. The pharmaceutical composition is also effective when applied to the skin of the torso of a patient, to the skin of the chest, stomach area, waist, hips or back, as is common in VZV infections or shingles.

It is also intended that the pharmaceutical composition may also contain ribonucleotides, siDNA/RNA chimeras, or can be combined with siRNAs. Furthermore, in a preferred embodiment of the invention the siDNA of the pharmaceutical composition can be applied using a transducing agent selected from the group comprising: the virus itself, a replicating Herpes virus particle which carries the siDNA into the cell during the process of infection, a liposome, nanoparticle, transmembrane carriers or peptides.

The invention also relates to siDNA oligonucleotides according to the present invention for use as an antiviral therapeutic agent in the treatment and/or prevention of Herpes virus infection.

A further embodiment of the invention relates to the use of siDNA oligonucleotides according to the present invention in the manufacture of a pharmaceutical composition for use as an antiviral therapeutic agent for the treatment and/or prevention of Herpes virus infection.

The sequences of the present invention are presented in Table 1. Minor variations of these sequences produced through techniques known to one skilled in the art, such as mutation, substitution, inversion, or other modifications that serve to provide the same functions of the DNA sequences as are described herein, are considered to be included in the scope of the present invention.

**Table 1: Sequences of the present invention**

| Name | SEQ ID NO. | Sequence (5' - 3') | Gene name | DNA/ RNA |
|---|---|---|---|---|
| ODN 5 | 1 | GTTCTTTCGGGTCATGCTCTTGTAATTTTTTAA TAGACAATGACCACGTTCGGA | UL5 | siDNA |
| ODN 29 | 2 | TTTGGGTCTAATCAAGGCCATTCGGTTTTCCG GTTGGAATTGATTGCGAAAGTT | UL29 | siDNA |
| ODN 30 | 3 | CCGTTTTTTTCATCATTTTCCTCTGTTTTCAGTC GAAGTTGATGATGTTGTACC | UL30 | siDNA |
| ODN 48 | 4 | CCCGTTGGGATGGTGGGGATGTCCCTTTTCC CGAATCAACACCATAAAGTACCC | UL48 | siDNA |
| ODN 48G | 5 | CCCGTTGGGATGGTGGGGATGGCCCTTTTCC CGAATCAACACCATAAAGTACCC | UL48 | siDNA |
| - | 6 | UCCUAACGUGGUCAUUGUCUAUUAA | UL5 | RNA target |
| - | 7 | AACUUUCGCAAUCAAUUCCAACCGG | UL29 | RNA target |
| - | 8 | GGUACUUCAUCAUCAACUUCGACUG | UL30 | RNA target |
| - | 9 | GGGUACUUUAUGGUGUUGAUUCGGG | UL48 | RNA target |

### DETAILED DESCRIPTION OF THE INVENTION

Under the term "complementary" the following should be understood: A nucleic acid molecule that is capable of binding another nucleic acid molecule through Watson-crick base pairing. The "percentage complementarity" refers to the percentage of nucleotides within a complementary sequence that pair with the nucleic acid sequence to be bound. The term "partially complementary" means a complementarity between 40 and 60%.

The invention is based on the concept that a DNA strand has a thermodynamic preference to form an RNA-DNA hybrid over double-stranded DNA or double-stranded RNA. Thus the invention is based on the unexpected result that siDNA is of high preference (in contrast to siRNA) to form RNA-DNA hybrids; siDNA is therefore of advantage and a preferred object of the invention.

Furthermore, siDNA is preferred due to the fact that DNA is easier to synthesize and more stable than siRNA. siDNA is also superior to a simple antisense oligodeoxynucleotides, because it is more stable during uptake and inside the cell, and is therefore more effective.

Furthermore, siDNA oligonucleotides can be stabilized by base modifications. Such base modifications entail 2'-O-methylated nucleotides and phosphorothioates at the ends and in the central linker regions to protect against nucleases and to increase stability and longevity both in vivo and in pharmaceutical preparations. Other chemical modifications are also envisaged that may improve the stability of the siDNA oligonucleotides.

The siDNA oligonucleotides of the present invention are designed to bind to RNA from all viruses of the family *Herpesviridae.* For example, the similarity between HSV-1 and HSV-2 viruses is very high in genes of particular interest. Despite an approximate difference in 50% of overall DNA sequence content between the two viruses, the sequences in the UL29, UL5, UL30 and UL48 genes are identical or close to identical. The target sites between HSV-1 and HSV-2 are identical for UL29 and 96 % identical for UL5. The genes UL29, UL5, UL30 and UL48 are highly conserved between different members of Herpes viruses and therefore are also intended targets in other viruses of the *Herpesviridae* family such as VZV, EBV, CMV and KSHV.

Due to the high sequence conservation of the UL29, UL5, UL30 and UL48 genes between different viruses of the *Herpesviridae* family, siDNA oligonucleotides of the present invention, especially those defined by SEQ ID NO. 1, SEQ ID NO. 2, SEQ ID NO. 3, SEQ ID NO. 4 and SEQ ID NO. 5, can be used to target the RNA, preferably corresponding to the UL29, UL5, UL30 and UL48 genes, of all viruses in the *Herpesviridae* family. Therefore, it is intended that the siDNA oligonucleotides of the present invention can be applied in the treatment or prevention of all Herpes virus infections.

Prior infection with another sexually transmitted disease (STD), such as HSV-2, predisposes an individual to a higher risk of contracting HIV. In the U.S. alone, up to 50% of the population is seropositive for HSV-2. This figure rises to 80% in sub-Saharan Africa, where HIV is endemic. Viral reactivation of HSV occurs in >95% of healthy individuals and leads to the formation of genital ulcerations, resulting in destruction of the mucosal barrier and an influx of activated CD4+ T cells-optimal conditions for HIV infection. Therefore, decrease in HSV-2 infection could lead to a significant drop in HIV infection rates.

Since vaccines giving mucosal protection are probably many years away and condoms have failed to become generally accepted by males in many parts of the world, protective means are required which are under the control of the woman and can, if necessary, be used without the knowledge or consent of the male partner. Vaginal microbicides meet this requirement and could not only protect the female's reproductive tract against infectious agents transmitted by the male, but could also protect the male's genital mucosa against possible infectious agents from the female.

Therefore it is a preferred embodiment of the present invention that vaginal microbicides are subject matter of the invention. In one embodiment, the siDNA ODN is formulated with a pharmaceutically acceptable carrier to form a microbicidal composition that can treat or prevent viral infection by a virus noted above. This is especially relevant for infections by HSV-1 or HSV-2. It is further intended that the siDNA ODNs of the present invention can be applied topically. Preferably, the microbicide is formulated for topical, particularly genital or rectal, administration, more preferably, for vaginal administration.

The administration of the siDNA oligonucleotides according to the present invention can occur in the prevention or in the treatment of Herpes virus infections. The mechanism that leads to destruction of the virus can occur in cells that have already been infected, in cells where viral DNA has first entered the cell or in viral particles pre-infection. The preferred effect is the transfection of siDNA oligonucleotides into infected cells, as is demonstrated in the examples. Also demonstrated is that when the siDNA is administered before viral infection, viral infection rates are subsequently significantly reduced, thus providing evidence for the preventative effect of the siDNA oligonucleotides of the present invention.

As discussed above, the oligonucleotides of the invention may be used in a pharmaceutical composition when combined with a pharmaceutically acceptable carrier. The term "pharmaceutically acceptable" means a non-toxic material that does not interfere with the effectiveness of the biological activity of the active ingredient(s). The characteristics of the carrier will depend on the route of administration. Such a composition may contain, in addition to the oligonucleotide and carrier, diluents, fillers, salts, buffers, stabilizers, solubilizers, and other materials well known in the art.

The pharmaceutical composition of the invention may also contain other active factors and/or agents which enhance inhibition of virus production by infected cells. For example, combinations of oligonucleotides, each of which targets a different Herpes virus gene or species, may be used in the pharmaceutical compositions of the invention. The pharmaceutical composition of the invention may further contain nucleotide analogs such as azidothymidine, dideoxycytidine, dideotyinosine, and the like. Such additional factors and/or agents may be included in the pharmaceutical composition to produce a synergistic effect with the oligonucleotide of the invention, or to minimize side-effects caused by the oligonucleotide of the invention.

The pharmaceutical composition of the invention may be in the form of a liposome in which the oligonucleotides of the invention is combined, in addition to other pharmaceutically acceptable carriers, with amphipathic agents such as lipids which exist in aggregated form as micelles, insoluble monolayers, liquid crystals, or lamellar layers which are in aqueous solution. Suitable lipids for liposomal formulation include, without limitation, monoglycerides, diglycerides, sulfatides, lysolecithin, phospholipids, saponin, bile acids, and the like. Preparation of such liposomal formulations is within the level of skill in the art.

The pharmaceutical composition of the invention may further include compounds which enhance delivery of oligonucleotides into cells. The oligonucleotides may be applied to using a transducing agent selected from the group comprising: the virus itself, a replicating Herpes virus particle which carries the siDNA into the cell during the process of infection, a liposome, nanoparticle, transmembrane carriers or peptides.

As used herein, the term "therapeutically effective amount" means the total amount of each active component of the pharmaceutical composition or method that is sufficient to show a meaningful patient benefit, e.g., reduction or healing of conditions characterized by Herpes virus infection and associated infections and complications or by other viral infections or increase in rate of healing of such conditions.

Such conditions can be cold sores, oral Herpes, small, painful, fluid-filled blisters or sores that appear on the lips, mouth, throat, cheek, chin, or nose. Genital Herpes refer to recurrent clusters of vesicles and lesions at the genital areas or below the waist.

When applied to an individual active ingredient, administered alone, the term refers to that ingredient alone. When applied to a combination, the term refers to combined amounts of the active ingredients that result in the therapeutic effect, whether administered in combination, serially or simultaneously.

In practicing the method of treatment or use of the present invention, a therapeutically effective amount of one or more of the oligonucleotide of the invention is administered to a mammal infected with Herpes virus. The oligonucleotide of the invention may be administered in accordance with the method of the invention either alone or in combination with other therapies such as treatments employing cytokines, lymphokines, other hematopoietic factors, other anti-viral agents, and the like.

Administration of the oligonucleotide of the invention used in the pharmaceutical composition or to practice the method of the present invention can be carried out in a variety of conventional ways, such as topical application, oral ingestion, inhalation, or cutaneous, subcutaneous, or intravenous injection. Topical application is preferred, especially via a microbicide.

A microbicide can be produced in many forms, including gels, creams, suppositories, films, douche, enema or as a sponge or ring that releases the active ingredient over time. Microbicides can offer both primary protection in the absence of condoms and back-up protection if a condom breaks or slips off during intercourse.

When a therapeutically effective amount of oligonucleotide of the invention is administered via topical application, the oligonucleotide will be in the form of a solution, lotion, gel, cream, spray, film or oil, with or without tissue penetration enhancing agents. Lubricating agents, such as magnesium stearate, are also typically added. When administered in liquid form, a liquid carrier such as water, petroleum, oils of animal or plant origin such as peanut oil, mineral oil, soybean oil, sesame oil, or synthetic oils may be added. The liquid form of the pharmaceutical composition may further contain physiological saline solution, dextrose or other saccharide solution, or glycols such as ethylene glycol, propylene glycol or polyethylene glycol. When administered in liquid form, the pharmaceutical composition contains from about 0.5 to 90% by weight of the oligonucleotide and preferably from about 1 to 50% oligonucleotide.

When a therapeutically effective amount of oligonucleotide of the invention is administered orally, the oligonucleotide will be in the form of a tablet, capsule, powder, solution or elixir. When administered in tablet form, the pharmaceutical composition of the invention may additionally contain a solid carrier such as a gelatin or an adjuvant. The tablet, capsule, and powder contain from about 5 to 95% oligonucleotide and preferably from about 25 to 90% oligonucleotide.

When a therapeutically effective amount of oligonucleotide of the invention is administered by intravenous, cutaneous or subcutaneous injection, the oligonucleotide will be in the form of a pyrogen-free, parenterally acceptable aqueous solution. The preparation of such parenterally acceptable solutions, having due regard to pH, isotonicity, stability, and the like, is within the skill in the art. A preferred pharmaceutical composition for intravenous, cutaneous, or subcutaneous injection should contain, in addition to the oligonucleotide, an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical composition of the present invention may also contain stabilizers, preservatives, buffers, antioxidants, or other additives known to those of skill in the art.

The amount of oligonucleotide in the pharmaceutical composition of the present invention will depend upon the nature and severity of the condition being treated, and on the nature of prior treatments which the patient has undergone. Larger doses of oligonucleotide may be administered until the optimal therapeutic effect is obtained for the patient, and at that point the dosage is not increased further. It is contemplated that the various pharmaceutical compositions used to practice the method of the present invention should contain about 1 ng to about 100 mg of oligonucleotide per kg body weight.

### FIGURES

The invention is further described by the figures. These are not intended to limit the scope of the invention.
- Fig 1.: Model for ODN-mediated inhibition of HSV-1 and the specific ODN sequences used.
- Fig 2.: Inhibition of HSV-1 by ODNs.
- Fig 3.: Analysis of ODN-mediated reduction of HSV-1 titer in Vero cells and primary lung fibroblasts.

### DETAILED DESCRIPTION OF THE FIGURES

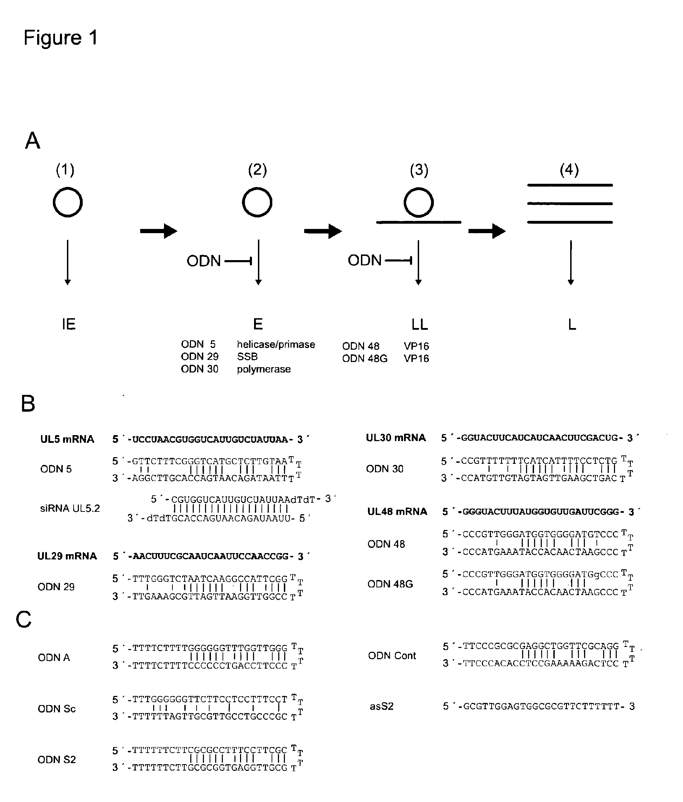

Fig 1. A) The HSV expression cascade during replication consists of the expression of (1) immediate early genes (IE) and (2) early (E) genes from the circularized HSV genome, of (3) leaky late genes (LL) from the parental genome and progeny genomes and of (4) late genes from progeny genomes. ODNs target mRNAs of early and leaky late gene products, a component of the helicase/primase complex, the single strand binding protein (SBB), the polymerase as well as the tegument protein VP16, which is a component of the virion and facilitates IE gene expression in the next round of infection. B) Target sites (bold) and sequences of the oligonucleotides used. C. ODN A, S2, Sc and Cont and asS2 served as negative controls.

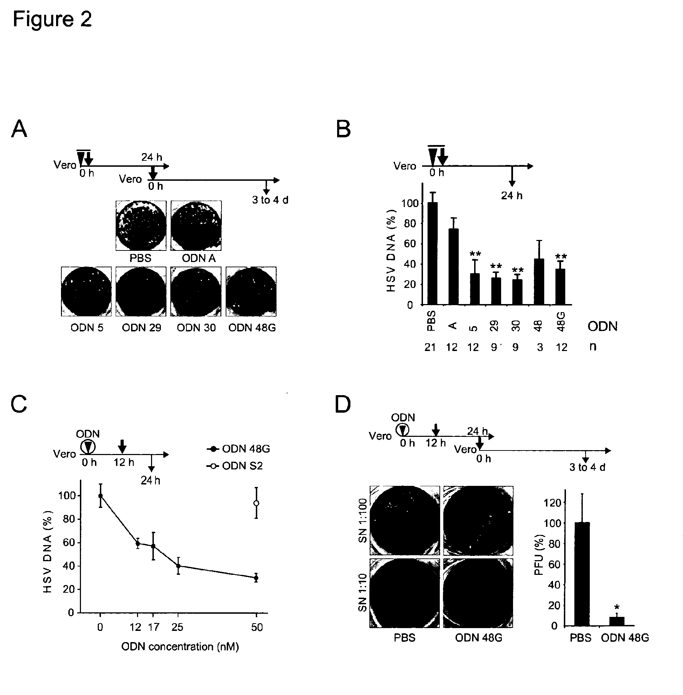

Fig 2. A) and B) Co-application of ODNs and HSV-1. HSV-1 virions were mixed with 2.5 µM of the indicated ODNs, a concentration needed in the absence of transfection reagent. The mixture was added to Vero cells (arrow head and bold arrow with overline). The titer of HSV-1 was determined after 24 h. A. Plaque assay. The supernatants of infected cultures were assayed for plaque formation on Vero cells. Representative 1:100 dilutions of triplicates are shown. B) Quantitative PCR. HSV-1 DNA was purified from supernatants and quantified by glycoprotein G-specific PCR. Relative HSV-1 DNA levels are shown +SE. The total number of infections (n) for each ODN is indicated below the graph. The statistical significance is indicated by asterisks, **P " 0.01 against PBS. C) Vero cells were treated with various concentrations of ODN 48G and S2 as indicated in the presence of transfection reagent (indicated by circle) at the indicated concentrations and were incubated over night (o/n) at 37°C. The cells were infected (bold vertical arrow) at a moi = 0.001 and after 24 h the HSV-1 DNA levels in the supernatants were measured by quantitative PCR (vertical arrow). The mean value ±SE of 2 different experiments is shown. D) Plaque Assay. Vero cells were transfected with 50 nM ODN 48G or phosphate-buffered saline (PBS) and infected as described in A. Dilutions of the supernatant were assayed for plaque formation on Vero cells. Representative pictures of triplicates are shown. *P " 0.05 against PBS.

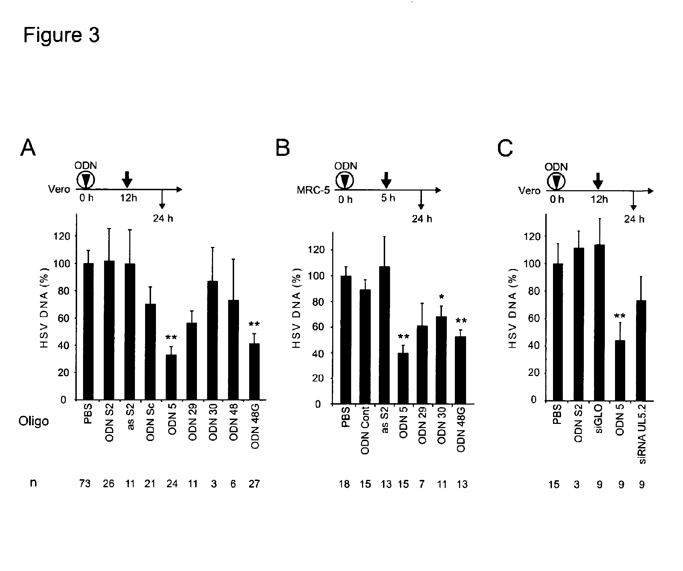

Fig 3. A), B) and C) Vero cells (A, C) and primary lung fibroblasts (B, MRC-5 cells) were treated with 50 nM of the indicated ODNs or PBS for 5 to 16 h in the presence of transfection reagent. The medium was changed to DMEM and cells were incubated for 1 h. The cells were infected and after 24 h the HSV-1 DNA levels in the supernatants were determined by quantitative PCR. The mean value of all experiments +SE is shown. The total number of infections performed (n) is indicated below the graph. **P " 0.01, *P " 0.05 against PBS. C) Comparison of ODN 5 and siRNA UL5.2 targeting the same region of UL5 mRNA, performed as described in A. As negative control the commercially available siRNA siGLO RISC-Free (Dharmacon) was used.

### EXAMPLES

The following examples illustrate the concept and feasibility of the present invention, but are not meant to limit the scope of the invention since alternative methods may be utilized to obtain similar results.

HSV genes are expressed in a highly regulated cascade during productive infection. They are grouped in immediate early (IE), early (E), leaky-late (LL) and late genes according to the time point of expression after infection (Figure 1A). The subject matter of the present invention relates to partially double-stranded hairpin loop-structured ODNs, with one strand completely complementary to the target mRNA, that can reduce Herpes virus replication *in vitro.* The siDNA oligonucleotides of the present invention have been designed to inhibit Herpes virus replication. Three of them are directed against early and two against leaky late genes. The sequences of the antisense strands of the ODNs used here were chosen on the basis of known siRNA sequences, recently shown to significantly inhibit HSV replication (Zhang YQ et al., Clin Exp Dermatol 2008, 33:56-61 and Palliser D et al., Nature 2006, 439:89-94).

The structure, sequences, and target sites of the ODNs are shown in figure 1B. ODN 5, 29, and 30 target the mRNA of the E genes *UL* 5, *UL* 29, and *UL* 30 essential for DNA synthesis. *UL* 5 encodes a component of the helicase/primase complex, *UL* 29 a single strand binding protein and *UL* 30 the DNA polymerase. ODN 48 and 48G target the mRNA of the LL gene *UL* 48, which encodes the virion associated VP16 needed in progeny virions for the transcriptional activation of IE genes in the next round of infection.

As controls, we used different oligodeoxynucleotides without any sequence similarity to any Herpes virus genome (figure 1 C) or phosphate buffered saline (PBS). The ODNs used were protected against nucleases by thioate modifications of the three terminal nucleotides at each end and in the T4 linker as described.

African green monkey kidney (Vero) cells and human embryonic lung fibroblasts (MRC-5) were chosen to demonstrate the effect of the ODNs on HSV type 1 (HSV-1) and type 2 (HSV-2) replication *in vitro.* Both cell lines are permissive to HSV strain McIntyre infection. Vero cells are defective in interferon production and HSV infections of monolayers produce a clear plaque phenotype. The MRC-5 cells were chosen to confirm the results in a primary human cell line.

### Example 1: Antiviral effect of ODNs in Vero cells

The media were replaced by Dulbecco's modified Eagle Medium (DMEM) with 2 % fetal calf serum (FCS) containing a mixture of HSV-1 virions and ODNs for co-application. The virus was introduced at a multiplicity of infection (moi) of 0.001 and the final concentration of ODNs was 2.5 µM. After 24 h the cell culture supernatants were harvested and HSV DNA levels were determined by quantitative PCR (qPCR) using primers and a probe targeting the HSV-1 glycoprotein G.

The ODNs against HSV-1 reduced the viral titer by 60 to 80 % in comparison to infected cells incubated with PBS. To confirm this result the supernatants from this experiment were serially diluted and used to infect confluent Vero cells for plaque assays (figure 2A). After 1 h of incubation with the virus the cells were overlaid with DMEM containing 2 % FCS and 0.4 % noble agar and grown for 3 to 4 days at 37°C. After removal of the medium the cells were washed with PBS and stained with 1% crystal violet in 20% ethanol.

The ODNs against HSV-1 exhibited a significant reduction of the viral titer, whereas the observed effect with the control ODN A was comparable to the infected cells treated with PBS as negative control. Several experiments were performed with co-application under the same conditions and measured the viral titer in cell culture supernatants by qPCR 24 h after co-application. The result is summarized in figure 2B. A two-tailed Student's t-test with unequal variance was used to perform statistical analysis. The reduction of viral titers was statistically significant for ODNs 5, 29, 30 and 48G (p < 0.01 against PBS), but not for ODN 48 and the control ODN A. The HSV-1 DNA levels could be reduced by 75 %, although HSV replication was slightly reduced by the control ODN A in comparison to cells that were infected without ODNs.

### Example 2: Transfection of ODNs using transfection reagents

In order to examine the effect in more detail, experiments were performed in Vero and MRC-5 cells using transfection reagents, in contrast to the above-mentioned experiment. To establish the best conditions for transfection, transfection rates were determined for a fluorescein-labelled ODN A with different transfection reagents in Vero cells. The best transfection rates were achieved after an overnight incubation with Lullaby or Dreamfect Gold transfection reagents, which were consecutively used in further experiments.

Vero cells were transfected with different concentrations of ODN 48G and incubated them overnight. Cells were subsequently infected with a moi of 0.001. Cells treated with the unspecific ODN S2 at a final concentration of 50 nM or PBS served as controls. 24 h after infection the HSV DNA levels in cell culture supernatants were determined by qPCR. The reduction of viral replication was dose dependent with a plateau at a concentration of 50 nM (figure 2C). The control ODN S2 did not show a reduction of viral titer at this concentration. The supernatants of the cells treated with ODN 48G at a concentration of 50 nM and the cells treated with PBS were used for plaque assays. A reduction of plaque forming units by 90 % in comparison to cells treated with PBS was observed (figure 2D). Transfection reagents thus allow a reduction of the ODN concentrations.

Further transfection studies were carried out with ODNs at 50 nM concentrations. Figure 3A displays a summary of all experiments with transfection of ODNs into Vero cells and subsequent infection after 12 h. The control oligonucleotides, single-stranded asS2, and scrambled ODN Sc were tested additionally to ODN S2 to investigate the sequence specificity of the observed effects. Only ODN Sc showed a slight reduction of the HSV-1 DNA levels, but this effect was not significant. ODN 5 reduced the HSV-1 DNA levels significantly by 70 % (p < 0.01) and ODN 48G by 60 % (p < 0.01). The ODNs 29, 30 and 48 did not show a significant reduction of the titer. A cytotoxic effect of the ODNs was not observed, as revealed by proliferation assays.

### Example 3: Antiviral effect of ODNs in MRC-5 cells

MRC-5 cells were transfected with ODNs at a final concentration of 50 nM. The cells were infected 5 h post transfection at a moi of 0.001 for 1 h and the viral titer was assayed 24 h after infection by qRT-PCR. HSV-1 replication was only impaired in cells treated with the HSV-specific ODNs (figure 3B). The control ODNs Cont and asS2 were negative. The effect was statistically significant for ODN 5, 30 and 48G. The ODNs did not exhibit a cytotoxic effect in MRC-5 cells.

### Example 4: Direct comparison between ODN and siRNA

A direct comparison between ODN5 and an siRNA is shown for Vero cells in Fig 3C. In a direct comparison with siRNAs and ODNs in Vero cells both oligonucleotides were differentially effective, e.g. the most effective ODN 5 in this study was 2.5-fold more effective than the corresponding siRNA-UL5.2 (Palliser D et al., Nature 2006, 439:89-94) (see figure 3C), whereas ODN 29 was 2-fold less effective than its analog.

### Example 5: Antiviral activity of ODNs on HSV-2 in MRC-5 cells

In order to demonstrate the effectiveness of siDNAs against other members of the Herpes virus family, we carried out experiments with HSV-2 in MRC-5 cells using transfection reagents as described above. MRC-5 cells were transfected with ODNs at a final concentration of 50 nM. The cells were infected 5 h post transfection with HSV-2 at a moi of 0.001 for 1 h and the viral titer was assayed 24 h after infection by qRT-PCR. HSV-2 replication was only impaired in cells treated with the Herpes-specific ODNs. As for HSV-1, the control ODNs Cont and asS2 were negative.

Table 2 summarizes all experiments listed under the different conditions and shows the reduction of HSV-1 and HSV-2 DNA levels upon treatment with ODNs in Vero and MRC-5 cells. Overall, ODN 5, targeting a component of the helicase/primase complex, has the greatest potential to inhibit HSV-1 and HSV-2 replication *in vitro,* which is consistent with reports about the helicase/primase complex being a target for inhibiting the HSV-1 replication by siRNA (Palliser D et al., Nature 2006, 439:89-94, see Fig 3C).

**Table 2: Reduction of HSV-1 and HSV-2 DNA levels upon treatment with ODNs in Vero and MRC-5 cells. The data of all in vitro experiments are summarized. The average fold reductions of the mean and the median are shown (n. d., not determined).**

| Treatment | Average fold reduction of the mean (median) | | | |
|---|---|---|---|---|
| | Co-application HSV-1 in Vero | Transfection with subsequent infection | | |
| | | HSV-1 in Vero | HSV-1 in MRC-5 | HSV-2 in MRC-5 |
| PBS | 1.0 (1.0) | 1.0 (1.0) | 1.0 (1.0) | 1.0 (1.0) |
| Control | 1.4 (1.4) | 1.0 (1.0) | 1.1 (1.1) | 1.1 (1.2) |
| ODN5 | 3.3 (6.5) | 3.0 (5.0) | 2.5 (2.2) | 2.6 (2.4) |
| ODN29 | 3.9 (4.1) | 1.8 (1.3) | 1.6 (2.4) | 1.6(2.1) |
| ODN30 | 4.1 (4.1) | 0.7 (1.3) | 1.5 (1.6) | 1.4 (1.5) |
| ODN48 | 2.2 (3.1) | 1.4 (1.7) | n. d. | 1.5 (1.4) |
| ODN48G | 2.9 (3.6) | 2.4 (2.4) | 1.9 (2.0) | 2.0(2.1) |

## Claims

1. Short interfering DNA oligonucleotides (siDNA) capable of binding to Herpes virus RNA, whereby the siDNA oligonucleotides comprise of an antisense-strand that is complementary to the Herpes virus target RNA and a second strand that is partially complementary to the antisense-strand, **characterized in that**
- the second strand is connected to the antisense strand by a thymidine linker, preferably 4 nucleotides in length,
- the antisense and second strands are 20 to 35 nucleotides in length, preferably 25 nucleotides in length,
- the antisense strand is more than 80% complementary, preferably more than 90% complementary, to the Herpes virus target RNA, and
- the second strand is 40 - 60% complementary to the antisense-strand and is capable of forming triple helices by non-Watson-Crick base pairing with the Herpes virus target RNA.

2. siDNA oligonucleotides according to claim 1, whereby the siDNA is stabilized by base modifications, preferably by phosphorothioate modification of the DNA.

3. siDNA oligonucleotides according to claims 1 or 2, comprising the DNA sequence of SEQ ID NO. 1, SEQ ID NO. 2, SEQ ID NO. 3, SEQ ID NO. 4 and/or SEQ ID NO. 5.

4. siDNA oligonucleotides according to one or more of the preceding claims, whereby the antisense strand of the siDNA binds Herpes virus RNA corresponding to the UL5, UL29, UL30 and/or UL48 genes.

5. siDNA oligonucleotides according to one or more of the preceding claims, whereby the antisense strand of the siDNA binds Herpes virus target RNA sequences selected from the group comprising SEQ ID NO. 6, SEQ ID NO. 7, SEQ ID NO. 8 and/or SEQ ID NO. 9.

6. siDNA oligonucleotides according to one or more of the preceding claims, whereby the target RNA is from a Herpes virus selected from the group comprising: HSV-1, HSV-2, VZV, EBV, CMV, Roseolovirus and/or KSHV.

7. Pharmaceutical composition comprising siDNA oligonucleotides, preferably according to claims 1 to 6, capable of binding to Herpes virus RNA as an antiviral therapeutic in the treatment and/or prevention of Herpes virus infection, whereby one or more of said siDNA oligonucleotides may be combined in a cocktail.

8. Pharmaceutical composition according to claim 7, whereby the siDNA oligonucleotide is combined with a pharmaceutically acceptable carrier to form a microbicidal composition for the treatment and/or prevention of Herpes virus infection.

9. Pharmaceutical composition according to claim 8, whereby the microbicidal composition can be applied topically, preferably to the lips, mouth, nose, chin, hips, back, waist and/or rectally or to the genitals, preferably to the vagina.

10. Pharmaceutical composition according to one of claims 7 to 9, whereby the composition may also contain ribonucleotides, siDNA/RNA chimeras, or can be combined with siRNAs.

11. Pharmaceutical composition according to one of claims 7 to 10, whereby the pharmaceutical composition comprises a transducing agent for the siDNA selected from the group comprising: the virus itself, a replicating Herpes virus particle which carries the siDNA into the cell during the process of infection, a liposome, nanoparticle, transmembrane carriers or peptides.

12. siDNA oligonucleotides according to claim 1 for use as an antiviral therapeutic agent in the treatment and/or prevention of Herpes virus infection.

13. Use of siDNA oligonucleotides according to claim 1 in the manufacture of a pharmaceutical composition for use as an antiviral therapeutic agent for the treatment and/or prevention of Herpes virus infection.
